# EUROPEAN PATENT SPECIFICATION

(11) **EP 1 882 483 B1**
(45) Date of publication and mention of the grant of the patent: **16.03.2011**
(21) Application number: 07252973.8
(22) Date of filing: 27.07.2007
(51) Int. Cl.: A61L 27/14, A61L 27/16, A61L 27/50, A61L 27/18

(54) **Medical implant bearing material**
Material zum Tragen eines medizinischen Implantats
Matériau de revêtement d'implant médical

(30) Priority: 28.07.2006 US 494932
(43) Date of publication of application: 30.01.2008
(73) Proprietor: DePuy Products, Inc., Warsaw, IN 46581 (US)
(72) Inventor: Ernsberger, Craig, IN 46530 (US); Valint, Paul, NY 14534 (US); Salvati, Larry, IN 46526 (US); Liao, Yen-Shuo, IN 46582 (US)
(74) Representative: Belcher, Simon James

(56) References cited:
- EP-A- 1 839 685
- WO-A-2004/032987
- WO-A-2004/071451
- MORO T ET AL: "Surface grafting of artificial joints with a biocompatible polymer for preventing periprosthetic osteolysis" 2004, NATURE MATERIALS, NATURE PUBLISHING GROUP, LONDON, GB, PAGE(S) 829-836 , XP002457808 ISSN: 1476-4660 * page 829 and figure 2 *

## Description

This invention relates to a bearing material for a medical implant.

Medical implants employ a polymeric material such as ultrahigh molecular weight polyethylene (UHMWPE) as the bearing material which articulates against a hard counterface, for example, a metallic counterface, of the implant. The polymeric material, however, tends to wear during use with concomitant production of wear debris comprising microscopic particles of the polymer. These particles can cause adverse reactions such as inflammation and deterioration of cell tissues, or osteolysis of the tissues.

Attempts have been made to reduce the wear rate of the bearing material, particularly UHMWPE, for example, by modifying the bulk properties of the polymer. Approaches to modify the bulk properties of the bearing material include radiation crosslinking of the polymer and stabilizing the associated free radicals against oxidation. While highly crosslinked UHMWPE has wear rates lower than the uncrosslinked material, the highly crosslinked material also tends to produce finer wear debris with higher osteolytic potential.

The foregoing shows that there exists a need for a medical implant or medical implant part, such as the bearing material, which has a reduced wear rate.

Moro et al (Nature Materials, Vol 3, pages 829 to 836) disclose polyethylene components of joint prostheses, in which phospholipid is grafted on to the surface of the polyethylene components.

In one aspect, the present invention provides a bearing material of a medical implant, as defined in claim 1.

In another aspect, the invention provides method of making a bearing material for articulation with a hard counterface in an orthopaedic joint prosthesis, as defined in claim 6.

The bearing material has one or more advantages, for example, a reduced wear rate, reduced amount of wear debris production, and/or reduced osteolytic potential. The surface active agent can act as a boundary lubricant and reduce the friction between the polymer of the bearing material when it articulates against the hard counterface. The bearing material of the invention made of UHMWPE and containing a surface agent can provide a lower wear rate than a bearing material made of crosslinked UHMWPE.

The surface active agent can be anionic, cationic, non-ionic, or amphoteric. The surface active agent can comprise both hydrophilic and hydrophobic moieties or segments. In one embodiment, the surface active agent can be a block copolymer. In another embodiment, the surface active agent can include an alkoxylated hydrophilic moiety and a hydrophobic moiety. The hydrophilic moiety can include one or more ethoxyl groups. The hydrophobic moiety can include one or more alkanol, alkenol, aromatic alcohol, alkylamine, alkenyl amine, alkyl aromatic alcohol, alkanoic acid, alkenoic acid, or any combination thereof.

The present invention arises from the observation that surface active agents reduce the friction, and/or provide a lubricating effect, between a polymer surface articulating against a hard counterface in a fluid medium such as serum containing a protein. Accordingly, in an embodiment, the invention provides a bearing material of a medical implant comprising a polymer and a surface active agent that is not covalently bonded to the polymer, wherein the bearing material is adopted for articulating against a hard counterface of the medical implant.

The bearing material in accordance with the invention can be that of any suitable medical implant or medical implant part. Suitable medical implants or medical implant parts include, but are not limited to, the acetabular cup, the insert or liner of the acetabular cup, or trunnion bearings (e.g., between the modular head and the stem) of artificial hip joints, the tibial plateau, patellar button (patello-femoral articulation), and trunnion or other bearing components of artificial knee joints, the talar surface (tibiotalar articulation) and other bearing components of artificial ankle joints, the radio-numeral joint, ulno-humeral joint, and other bearing components of artificial elbow joints, the glenoro-humeral articulation and other bearing components of artificial shoulder joints, intervertebral disk replacements and facet joint replacements for the spine, temporo-mandibular joints (jaw), and finger joints.

As utilized herein, the term "ultrahigh molecular weight polyethylene" refers to a polyethylene polymer having a weight average molecular weight of about 400,000 atomic mass units or more. Preferably, the ultrahigh molecular weight polyethylene has a weight average molecular weight of about 1,000,000 (e.g., about 2,000,000 or about 3,000,000) atomic mass units or more. Typically, the weight average molecular weight of the ultrahigh molecular weight polyethylene is less than 10,000,000 atomic mass units or less, more preferably about 6,000,000 atomic mass units or less. Ultrahigh molecular weight polyethylene suitable for use in the invention includes, but is not limited to, commercially available ultrahigh molecular weight polyethylene, such as Resin Sold under trade mark GUR 1050 (weight average molecular weight of 5,000,000 to 6,000,000 atomic mass units) or Resin Sold under trade mark GUR 1020 (weight average molecular weight of 3,000,000 to 4,000,000 atomic mass units) powdered ultrahigh molecular weight polyethylene from Ticona (Summit, N.J.).

The surface active agent is an added (exogenous) surface active agent, and does not refer to any surface active agent that may be present in the synovial fluid naturally. The surface active agent can be of any suitable molecular weight, for example, from 100 to 20,000 g.mole⁻¹ or more, and in embodiments from 1000 to 20,000 g.mole⁻¹.

The nonionic surface active agent comprises an alkoxylated hydrophilic moiety, such as an ethoxylated moiety, and a hydrophobic moiety. An alkoxylated hydrophilic moiety can include from 3 to 110 alkylene oxide units, from 10 to 80 alkylene oxide units, or from 20 to 50 alkylene oxide units. For example, an alkoxylated hydrophilic moiety can include from 3 to 10, from 11 to 25, from 26 to 40, from 41 to 60, from 61 to 80, or from 81 to 110 alkylene oxide units. The hydrophobic moiety can include a moiety obtainable from an alkanol, alkenol, aromatic alcohol, alkylamine, alkenyl amine, alkyl aromatic alcohol, alkanoic acid, alkenoic acid, or any combination thereof. For example, the hydrophobic moiety can include a C₈-C₁₀₀ alkyl chain, such as a C₁₀-C₅₀ or a C₁₅-C₃₀ alkyl or alkenyl chain. In specific examples, the alkoxylated or ethoxylated fatty alcohol can include a C₈-C₁₁, C₁₂-C₁₄, C₁₃-C₁₅, C₁₆-C₁₈, C₁₉-C₂₀, C₂₁-C₂₃, C₂₄-C₂₆, C₂₇-C₃₀, C₃₁-C₃₅, C₃₆-C₄₀, C₄₀-C₄₅, or C₄₅-C₅₀, a C₅₀-C₅₅, C₅₅-C₆₀, C₆₀-C₆₅, C₆₅-C₇₀, C₇₀-C₇₅, C₇₅-C₈₀, C₈₀-C₈₅, C₈₅-C₉₀, C₉₀-₉₅ or C₉₅-C₁₀₀ alkyl or alkenyl chain.

Non-ionic surface active agents that include an alkoxylated hydrophilic moiety and a hydrophobic moiety may be purchased commercially. For example, BASF Corp. (Florham Park, N.J.) offers a number of products under the LUTENSOL trade mark. Ethoxylated fatty alcohols include LUTENSOL type A to N, (e.g., type A4N, A7N, A79N, and A8N) and LUTENSOL type AT, e.g., types AT 11, AT 18, AT 25, AT 50, and AT 80). Other surface active agents include ethoxylated oxo alcohols available as LUTENSOL types AO and TO, ethoxylated Guerbet alcohols available as LUTENSOL types XP and XL, ethoxylated alkyl phenol alcohols available as LUTENSOL type AP, ethoxylated alkyl amines available as LUTENSOL type FA. Baker Petrolite (Sugarland, TX) is another supplier of nonionic surface active agents, including a series of ethoxylated fatty acid alcohols supplied under the Unithox trade mark.

The surface active agent can have a hydrophilic-lipophilic balance (HLB) value of from 4 to 24, for example from 7 to 10, from 10 to 12, from 12 to 14, from 14 to 16, from 16 to 19 or from 19 to 24. For example, surface active agent can have an HLB value of approximately any one of the following nearest integers: 4, 5, 6, 7, 8, 9, 10, 11, 12, 13, 14, 15, 16, 17, 18, 19, 20, 21, 22, 23, or 24. HLB values can be calculated according to the system introduced by W.C. Griffin. See, e.g., "Classification of Surface-Active Agents by 'HLB,"' Journal of the Society of Cosmetic Chemists 1: 311 (1949), "Calculation of HLB Values of Non-Ionic Surfactants," Journal of the Society of Cosmetic Chemists 5: 259 (1954), and Encyclopedia of Chemical Technology Vol. 8, Wiley, New York (1965).

The surface active agent can be incorporated into the bearing material in any suitable amount, for example, in an amount of 0.01% by weight or more, e.g., from 0.1% to 5%, and preferably from 0.5% to 1% by weight of the bearing material.

The surface active agent can be included in the bearing material by any suitable method, for example, by molding, ram extrusion, or infiltration of the finished polymeric (e.g., UHMWPE) article with the surface active agent in a suitable solvent. Thus, for example, powdered or pelletized UHMWPE and a surface active agent can be blended, e.g., dry blended, at a desired concentration and the resulting blend can be molded by compression molding or ram extrusion. For example, UHMWPE and a surface active agent can be blended in an aqueous solution to form an aqueous dispersion containing a suitable amount of a surface active agent, e.g., more than 0.1%, from 0.1% to 5% or from 0.5% to 1% by weight of UHMWPE. The dispersion can be poured into a suitable tray and frozen at -80 °C. The tray can be loaded into a freeze dryer and a vacuum applied. The water can be removed by sublimation leaving a uniform dispersion of surface active agent and UHMWPE powder. This powder can be added to a mold and processed under temperature and pressure to produce a molded article.

The surface active agent can be originally distributed throughout the bearing material or only present on the small portion thereof, e.g., at the surface. Thus, for example, the surface active agent can be present in a surface layer having a thickness of 0.5 mm or more, e.g., from 0.5 mm to 2 mm or more. The internal volume of the compression mould can be filled entirely with a blend of the polymer and the surface active agent, or alternatively, a portion of the internal volume can be filled with the blend, and the remaining volume filled with the polymer. In the latter case, the surface active agent will be contained in the surface of the bearing material.

Alternatively, the bearing material can be immersed in a solution of the surface active agent at a suitable temperature and for suitable length of time to obtain a sufficient concentration of the surface active agent in the polymer. The solvent can be removed by suitable drying.

The surface active agent can be provided such that it elutes from a non-articulating portion of the medical implant.

Surface active agent present in the bulk of the polymer, it migrates to the interface between the polymer and the serum due to the reduction of surface energy.

The hard counterface of the implant can be made of any suitable material, e.g., metallic, ceramic, or a combination thereof. Suitable metals include titanium, tantalum, and stainless steel. Typically, the hard counterfaces are formed from a metallic alloy that will exhibit appropriate strength and flexure in use. Examples of metallic alloys that may be used include titanium alloys, such as a titanium-aluminum-vanadium alloy, and cobalt-chromium alloys, such as a cobalt-chromium-molybdenum alloy, and stainless steel. In a specific example, the hard counterface comprises cobalt-chromium-molybdenum alloy.

The invention is described below with reference to the accompanying drawings, in which:
Figure 1 depicts the known wear rate of UHMWPE bearing material against various counterfaces as a function of the concentration of proteins in the serum.
Figure 2A depicts the weight change of UHMWPE pin as it rubs against cast CoCrMo counterfaces in water and in bovine calf serum plus 1% by weight surface active agent in accordance with an embodiment of the invention.
Figure 2B depicts the weight change of the UHMWPE pins in bovine calf serum only.
Figure 3 depicts the coefficient of friction between UHMWPE pins and CoCrMo counterfaces as a function of the number of cycles in serum (diamonds), 1% by weight surface active agent (triangles), and serum plus 1% by weight surface active agent (x), in accordance with an embodiment of the invention.

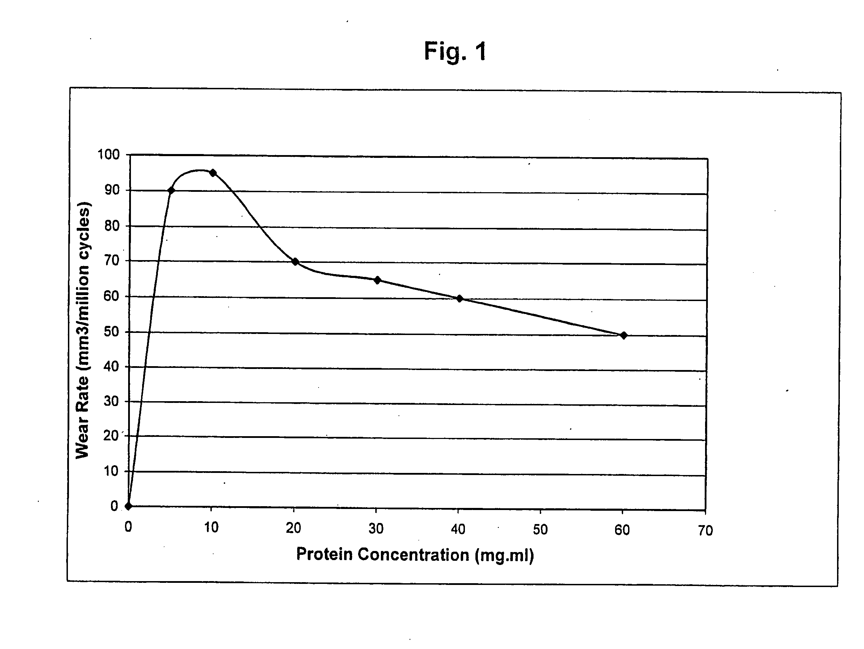

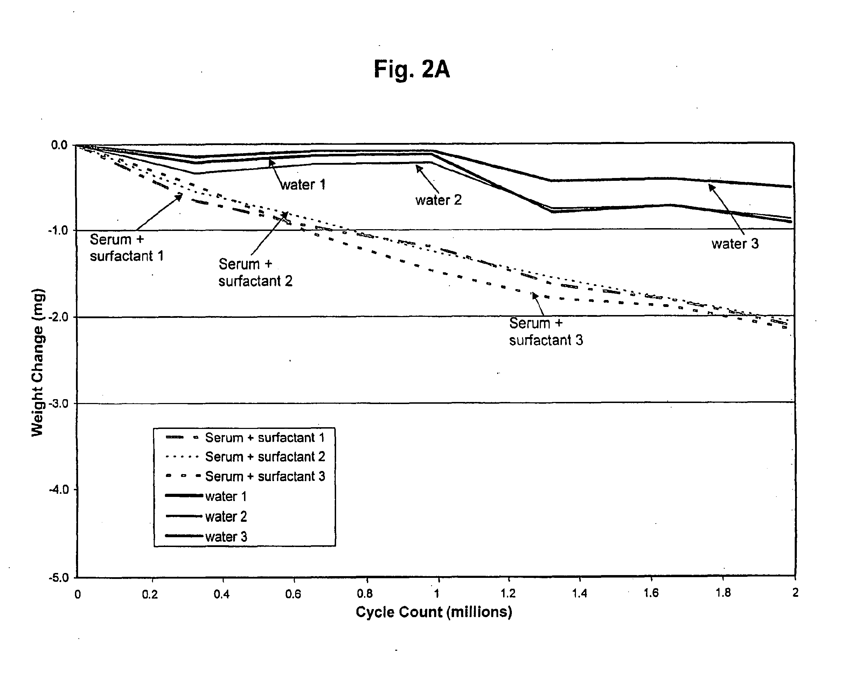

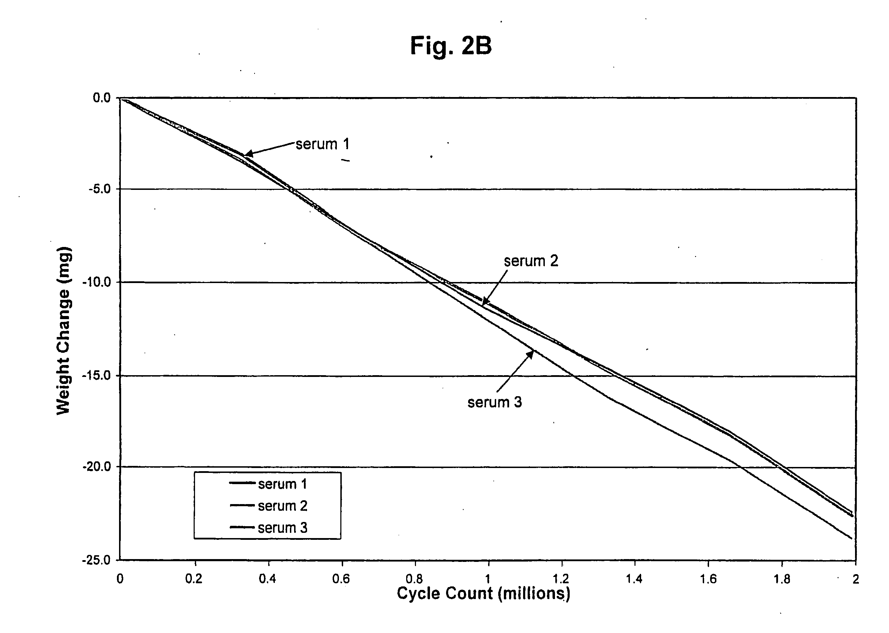

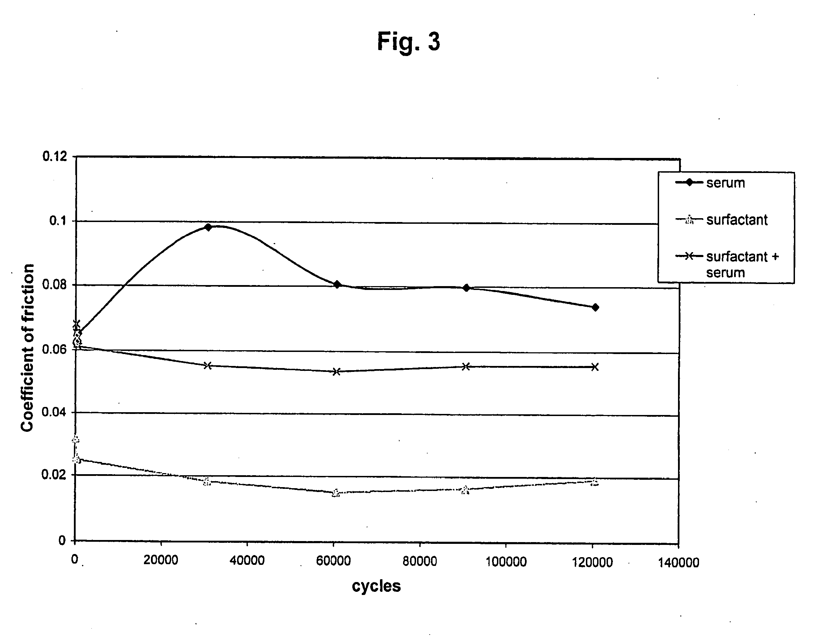

It is known that proteins tend to alter the wear rate of UHMWPE. For example, as shown in Fig. 1, the wear rate increases with increasing protein concentration up to a certain protein concentration. Beyond this concentration, the wear rate tends to decrease. In accordance with the present invention, by the use of a surface active agent, it is possible to reduce the wear rate even further. As depicted in Figure 2, the wear rate of UHMWPE in a serum containing 1% surface active agent is less than that in serum alone. Further, as shown in Figure 3, the coefficient of friction of UHMWPE against Co-Cr-Mo counterface is decreased by the use of a surface active agent in the serum.

### COMPARATIVE EXAMPLE 1

This example illustrates an advantage of the surface active agent in accordance with an embodiment of the invention, namely, it reduces the wear rate of UHMWPE.

UHMWPE pins, 18 mm (0.7 inch) long and 9.5 mm (0.375 inch) diameter, are manufactured from resin sold under trade mark GUR 1020, which is ram extruded into bar stock. The pins are sterilized by irradiation. Metal counterfaces, 38 mm (1.5 inch) diameter and 12.7 mm (0.5 inch) thick, are fabricated from cast 68% cobalt, 26% chromium, 6% molybdenum, and 0.2% carbon. The counterfaces are hot isostatic pressed and homogenized by heat treatment and polished to an average surface roughness of between 10 and 20 nm. Three disks are obtained for each wear test group. A sample pairing chart including same identifications is set out in the following table.

| Sample group | Pin | Disk | Station |
|---|---|---|---|
| | S1 | 2B | 1 |
| Serum and surface active agent | S2 | 5B | 2 |
| | S3 | 6 | 3 |
| | W1 | 7 | 4 |
| Water | W2 | 8 | 5 |
| | W3 | 9 | 6 |
| | 4 | 43 | 4 |
| Serum | 5 | 44 | 5 |
| | 6 | 45 | 6 |

The wear tests are conducted on a Pin-on-Disk (POD) machine (AMTI (Watertown, MA) OrthoPOD). The pins move in a 10 mm by 10 mm square pattern with respect to the disk, providing a maximum amount of cross shear motion. A Paul loading cycle with a peak of 330N is applied (Paul, J., Forces transmitted by joints in the human body, Proc. Inst. Mech. Eng., 181, 8-15 (1967)). The frequency is 1.6 Hz. Six, 0.33 million-cycle data collection intervals are performed for 1.98 million cycle test duration. Water lost by evaporation is replenished approximately every 24 hours. The test lubricant is discarded at the end of each test interval, and fresh lubricant is added.

After each data collection interval, each sample pair is rotated clockwise one station for the next interval. During the course of the test all of the samples are tested in all of the stations. Three different lubricants are tested: Reverse Osmosis treated water, Bovine serum from Hyclone Inc. (Logan, UT) (diluted to 90% of the initial concentration and treated with EDTA and sodium azide), and the bovine serum formulation above containing 1% by weight of a surfactant sold under the trade mark PLURONIC F127. Bulk serum temperature is maintained at 37 ± 1°C.

Gravimetric data is obtained from the pins as follows. The pins are cleaned and weighed in a microbalance with 0.01 mg resolution. The balance is calibrated with standard weights. Corrections for fluid uptake are applied using standard soak control pins. Contact profilometry of the disks is performed on a Taylor-Hobson Form Talysurf Series II Profilometer. A cut off length of 250 µm, a 100:1 1 bandwidth, and a gauge range of 1 mm are used. Two pairs of perpendicular traces are taken on each sample. Pictures of the pins are taken with a Nikon Epiphot 200/300 Inverted Microscope (metallograph). Pictures of the disks are taken with a Nikon D 1 Digital Camera.

Fig. 2A and 2B depict the wear rate of UHMWPE pins. The wear rate of the pins is less when a surface active agent is employed.

### COMPARATIVE EXAMPLE 2

This example demonstrates the reduction in the coefficient of friction between the polymer surface and the hard counterface.

Friction data is collected on the same type of pins and disks illustrated in Example 1. The data are obtained on an AMTI OrthoPOD Pin-on-Disk tester equipped with multiaxis strain gauges. The coefficient of friction is calculated by dividing the normal force by the in plane force during the same 10 mm by 10 mm test pattern and Paul load cycle used in wear testing. The friction data is collected over a small portion of the test pattern between the peaks of the Paul loading curve. Fig. 3 depicts the reduction in the coefficient of friction brought about by the use of the surfactant. Each point in Fig. 3 is the average of approximately 30 data points.

### EXAMPLE

This example illustrates a method of incorporating a non-ionic surface active agent into the UHMWPE.

UHMWPE is fabricated with one or both of two ethoxylated fatty alcohols having the nominal formulas C₃₈(EO)₁₁ and C₃₈(EO)₁₀₃. The ethoxylated fatty alcohols are available from Baker Petrolite (Sugarland, TX) in neat form (sold under the trade marks Unithox 450 with an HLB value of 10 and Unithox 490, with an HLB value of 18, respectively) or as aqueous dispersions (Unithox D100 and Petrolite D1038, respectively). The procedure begins by preparing an aqueous dispersion of UHMWPE powder (identified as GUR 1020 grade) with the D100 and/or D1038 ethoxylate at a level of 1% by weight of the UHMWPE material. Once the dispersion is prepared, it is poured into at least one suitable tray and frozen at -80 °C. The tray is then loaded into a freeze dryer and a vacuum is pulled on the frozen dispersion. The water is removed by sublimation, leaving a uniform dispersion of ethoxylate and UHMWPE powder. This powder is then added to a 89 mm (3.5 inch) diameter mould and processed under temperature and pressure to produce a moulded article.

## Claims

1. A bearing material of a medical implant comprising a polymer and a surface active agent which is not covalently bonded to the polymer, in which the bearing material can be used for articulating against a hard counterface of the medical implant and in which the polymer comprises ultrahigh molecular weight polyethylene (UHMWPE), **characterised in that** the surface active agent is an alkoxylated fatty alcohol which includes a C₁₅ to C₁₀₀ alkyl or alkenyl chain.

2. The bearing material of claim 1, in which the alkoxylated fatty alcohol includes from 3 to 110 alkylene oxide units.

3. The bearing material of claim 2, in which the alkoxylated fatty alcohol includes from 10 to 80 alkylene oxide units.

4. The bearing material of claim 2, in which the alkoxylated fatty alcohol includes from 20 to 50 alkylene oxide units.

5. A method of making a bearing material for articulation with a hard counterface in an orthopaedic joint prosthesis, which comprises:
blending a polymer and a surface active agent, and
moulding the blended polymer and surface active agent into a desired shape,
in which the surface active agent is not covalently bonded to the polymer in the moulded material, in which:
(a) the polymer comprises ultrahigh molecular weight polyethylene (UHMWPE), and
(b) the surface active agent is an alkoxylated fatty alcohol which includes a C₁₅ to C₁₀₀ alkyl or alkenyl chain.

## Patentansprüche

1. Lagermaterial fiir ein medizinisches Implantat, umfassend ein Polymer und eine oberflächenaktive Substanz, die nicht kovalent an das Polymer gebunden ist, wobei das Lagermaterial zur Artikulation gegen eine harte Gegenfläche des medizinischen Implantats verwendet werden kann und wobei das Polymer Polyethylen mit ultrahohem Molekulargewicht (UHMWPE) umfasst, **dadurch gekennzeichnet, dass** die oberflächenaktive Substanz ein alkoxylierter Fettalkohol ist, der eine C₁₅- bis C₁₀₀-Alkyl- oder -Alkenylkette einschließt.

2. Lagermaterial nach Anspruch 1, wobei der alkoxylierte Fettalkohol von 3 bis 110 Alkylenoxid-Einheiten einschließt.

3. Lagermaterial nach Anspruch 2, wobei der alkoxylierte Fettalkohol von 10 bis 80 Alkylenoxid-Einheiten einschließt.

4. Lagermaterial nach Anspruch 2, wobei der alkoxylierte Fettalkohol von 20 bis 50 Alkylenoxid-Einheiten einschließt.

5. Verfahren zur Herstellung eines Lagermaterials zur Artikulation mit einer harten Gegenfläche in einer orthopädischen Gelenkprothese, welches umfasst:
Vermischen eines Polymers und einer oberflächenaktiven Substanz und
Ausformen des Gemisches aus Polymer und oberflächenaktiver Substanz zu einer gewünschten Form, wobei die oberflächenaktive Substanz im ausgeformten Material nicht kovalent an das Polymer gebunden ist, wobei:
(a) das Polymer Polyethylen mit ultrahohem Molekulargewicht (UHMWPE) umfasst und
(b) die oberflächenaktive Substanz ein alkoxylierter Fettalkohol ist, der eine C₁₅-bis C₁₀₀-Alkyl- oder -Alkenylkette einschließt.

## Revendications

1. Matériau de support pour un implant médical comprenant un polymère et un agent tensioactif qui n'est pas lié de façon covalente au polymère, lequel matériau de support peut être utilisé pour une articulation contre une contre-surface dure de l'implant médical, et dans lequel le polymère comprend du polyéthylène de masse moléculaire ultra élevée (UHMWPE), **caractérisé en ce que** l'agent tensioactif est un alcool gras alcoxylé contenant une chaîne alkyle ou alcényle en C₁₅ à C₁₀₀.

2. Matériau de support selon la revendication 1, dans lequel l'alcool gras alcoxylé contient de 3 à 110 motifs oxyde d'alkylène.

3. Matériau de support selon la revendication 2, dans lequel l'alcool gras alcoxylé contient de 10 à 80 motifs oxyde d'alkylène.

4. Matériau de support selon la revendication 2, dans lequel l'alcool gras alcoxylé contient de 20 à 50 motifs oxyde d'alkylène.

5. Procédé pour préparer un matériau de support pour une articulation avec une contre-surface dure dans une prothèse d'articulation orthopédique, qui comprend :
■ le mélange d'un polymère et d'un agent tensioactif, et
■ le moulage du polymère et de l'agent tensioactif mélangés sous une forme souhaitée,
dans lequel l'agent tensioactif n'est pas lié de façon covalente au polymère dans le matériau moulé, dans lequel :
(a) le polymère comprend du polyéthylène de masse moléculaire ultra élevée (UHMWPE), et
(b) l'agent tensioactif est un alcool gras alcoxylé qui contient une chaîne alkyle ou alcényle en C₁₅ à C₁₀₀.
